# EUROPEAN PATENT SPECIFICATION

(11) **EP 1 603 527 B1**
(45) Date of publication and mention of the grant of the patent: **04.02.2009**
(21) Application number: 04719411.3
(22) Date of filing: 11.03.2004
(51) Int. Cl.: A61Q 13/00, C11B 9/00, A61K 8/31, A61K 8/362

(54) **TERPENOID FRAGRANCE COMPONENTS STABILIZED WITH MALONIC ACID SALTS**
MIT MALONSÄURESALZEN STABILISIERTE TERPENOID-DUFTKOMPONENTEN
COMPOSANTS PARFUMES A BASE DE TERPENOIDES STABILISES AVEC DES SELS D'ACIDE MALONIQUE

(30) Priority: 17.03.2003 US 455320 P
(43) Date of publication of application: 14.12.2005
(73) Proprietor: Unilever PLC, London EC4Y 0DY (GB); Unilever N.V., 3013 AL Rotterdam (NL)
(72) Inventor: SUBRAMANYAN, Krishna Kumar, Trumbull, CT 06611 (US); FARYNIARZ, Joseph Raymond, Middlebury, CT 06762 (US); ZHANG, Joanna Hong, Trumbull, CT 06611 (US)
(74) Representative: Acham, Nicholas Clive
(86) International application number: PCT/EP2004/002558
(87) International publication number: WO 2004/082652

(56) References cited:
- WO-A-00/61105
- WO-A-97/32569
- WO-A-03/099238
- WO-A-03/099250
- WO-A-03/099251
- WO-A-03/099252
- WO-A-03/099254
- DATABASE WPI Section Ch, Week 197908 Derwent Publications Ltd., London, GB; Class D22, AN 1979-14719B XP002285877 & JP 54 005041 A (TOKYO ORGANIC CHEM IND CO LTD) 16 January 1979 (1979-01-16)

## Description

### BACKGROUND OF THE INVENTION

The invention concerns personal care compositions fragranced with terpenoid components stabilized against degradation.

Terpenes are widespread in nature, mainly in plants as constituents of essential oils. Many terpenes are hydrocarbons, but oxygen-containing compounds such as alcohols, aldehydes or ketones (*terpenoids*) are also found. Their building block is the hydrocarbon isoprene, CH₂=C(CH₃)-CH=CH₂ (*isoprene* rule, Wallach 1887). Terpene hydrocarbons therefore have molecular formulas (C₅H₈)ₙ, they are classified according to the number of isoprene units: monoterpenes (n=2), sesquiterpenes (n=3), diterpenes (n=4), triterpenes (n=6) and tetraterpenes (n=8).

Examples of monoterpenes are: pinene, nerol, citral, camphor, geraniol and limonene. Examples of *sesquiterpenes* are: nerolidol and famesol. Squalene is an example of a *triterpene*.

The term "terpenoid", for purposes of the present invention, is intended to cover terpenes and oxygen containing derivatives thereof having at least one C₅H₈ hydrocarbyl unit which may have one or more points of unsaturation and/or be part of a cyclic unit within the chemical structure.

Terpenoids are a fragrance raw material of many perfume and scents. Fragrance terpenoids are disclosed in Arctander, S., Perfume and Flavor Chemicals, Vol. I and 11, Allured Pub. Co. (1969) and examples include Ambregris odour chemicals, Jasmones, Musks, Pyran-derivatives, and Sandalwood fragrance chemicals.

It is well known that mixtures of perfume or fragrance raw materials when deposited on a surface or when incorporated in a personal care composition lose intensity and may change character with time, due to many factors. Many attempts have been made to minimize these drawbacks, but so far with minimal success. For examples, Gardlick et al, U.S. Patent No. 6,147,037 relates to fragrance delivery systems useful in delivering sustained or enduring fragrances to personal care items.

The following documents are prior art under Article 54(3) EPC: WO 03/099250 A1 discloses a cosmetic cream comprising diammonium malonate, bisabolol and a fragrance; WO 03/099251 A1 discloses a topical composition comprising ammonium malonate, bisabolol and a fragrance; WO 03/099252 A1 discloses a topical composition comprising dimethylaminoethanol malonate, bisabolol and a fragrance; WO 03/099254 A1 discloses a cosmetic cream comprising a malonate salt bisabolol and a fragrance; and WO 03/099238 A1 discloses a cosmetic cream comprising diammonium malonate, bisabolol and a fragrance.

WO 97/32569 A1 discloses personal cleansing bar compositions which contain a fragrance-releasing complex and a bar carrier.

One factor responsible for loss of fragrance intensity or change in character is that terpenoids are oxidatively unstable. The degree of oxidative instability depends on the degree of unsaturation, or the number of double or triple bonds in the organic compound.

Oxidative instability is an undesirable characteristic in terpenoids. There is a need, therefore, for an agent that will stabilize terpenoids against oxidation. In particular, there is a need for an agent that will prevent the oxidation of terpenoids which have at least one double bond in its chemical structure.

### SUMMARY OF THE INVENTION

A personal care composition is provided which includes:
(i) from about 0.001 to about 10% of a fragrance by weight of the composition which includes from about 0.000001 to about 90% of a hydrocarbon cyclic terpenoid by weight of the fragrance;
(ii) from about 0.0001 to about 30% by weight of the composition of a salt of malonic acid; and
(iii) from about 1 to about 99% by weight of the composition of a cosmetically acceptable carrier.

### DETAILED DESCRIPTION OF THE INVENTION

We have now found that salts of malonic acid are effective at stabilizing terpenoid fragrance components against degradation.

A wide variety of counter cations to the malonate anions may be utilized in forming the salt. Malonate salts may either be the half or fully neutralized malonic acid or combinations thereof as represented by general formulas (I) and (II): 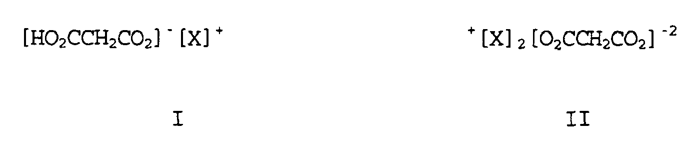 wherein X is a cationic counterion.

Suitable cationic counterions include those of alkali and alkaline earth metals. Representative examples include the cations of lithium, sodium, potassium, magnesium, calcium, ammonium and combinations thereof.

Not only inorganic but also organic cations can be employed. Particularly useful are quaternized nitrogen cations having from 1 to 1,000, preferably from 1 to 20, and optimally from 3 to 12 carbon atoms. Illustrative are those cations derived from amines which include triethanolamine, diethanolamine, propanolamine, monoethanolamine, methylamine, ethylamine, propylamine, isopropylamine, butylamine, isobutylamine, t-butylamine, pentylamine, isopentylamine, hexylamine, cyclohexylamine, cyclopentylamine, norbornylamine, octylamine, ethylhexylamine, nonylamine, decylamine, pyrrolidone, amino acids (lysine, arginine, alanine, glutamine, histidine, glycine), 2-amino-2-methyl-1-propanol, dimethylethanolamine, polyethyleneimine, tris(hydroxymethyl)amino methane and combinations thereof. Most preferred are the cations derived from ammonia, dimethylethanolamine and tris(hydroxymethyl)amino methane. Typical malonate salts derived from these preferred materials include ammonium malonate, diammonium malonate, dimethylethanolammonium malonate, bis(dimethylethanolammonium)malonate, tris(hydroxymethyl)methane ammonium malonate, and di[tris(hydroxymethyl)methane ammonium]malonate.

Amounts of the malonic acid salt may range from about 0.0001 to about 30%, preferably from about 0.1 to about 15%, more preferably from about 0.5 to about 10%, optimally from about 1 to about 8% by weight of the cosmetic composition.

The present invention can utilize as the active ingredient salt I, salt II or mixtures of these salts. When mixtures are present the molar ratio of mono-salt I to di-salt II may range from about 1000:1 to about 1:1000, preferably from about 10:1 to about 1:500, more preferably from about 2:1 to about 1:200, optimally from about 1:1 to about 1:20.

The term "personal care composition" is intended to describe products for topical application to human skin, including leave-on and wash-off products. Illustrative examples include skin creams and lotions, hair treatments such as shampoos, depilatories, shaving creams, antiperspirants and deodorants and shower gels.

The term "fragrance" is intended to mean one, but preferably two, or more fragrance raw materials which are artfully combined to impart a pleasurable scent, odor, essence, or fragrance characteristic.

As used herein, the term "unsaturation" is intended to describe an organic compound having double or triple bonds, such as olefin or an alkyne.

Terpene derivatives are known to be categorized in three classes which can include alcohols, ethers, aldehydes, acetals, acids, ketones, esters, and terpene compounds that contain heteroatoms such as nitrogen or sulfur.

Examples of terpenoids that may be incorporated in the cosmetic compositions of the present invention are hydrocarbon cyclic terpenoids set forth in the table below:

| HYDROCARBONS | |
|---|---|
| Limonene | (1,8-*p*-menthadiene) |
| Alpha-Terpinene | |
| Gamma-Terpinene | (1,4-*p*-menthadiene) |
| Terpinolene | |
| Alpha-Phellandrene | (1,5-*p*-menthadiene) |
| Beta-Phellandrene | |
| Alpha-Pinene | (2-pinene) |
| Beta-Pinene | (2(10)-pinene) |
| Camphene | |
| 3-Carene | |
| Caryophyllene | |
| (+)-Valencene | |
| Thujopsene | |
| Alpha-Cedrene | |
| Beta-Cedrene | |
| Longifolene | |

The preferred terpenoids are limonene, alpha-terpinene, gamma-terpinene, alpha-pinene and beta-pinene.

Further more, additional terpenoids can be incorporated into the personal cleansing composition as set forth in the tables below :

**TABLE 1. Acyclic Terpenoids**

| | |
|---|---|
| HYDROCARBONS | |
| Myrcene | |
| Ocimene | |
| beta-Farnesene | |

| | |
|---|---|
| ALCOHOLS | |
| Dihydromyrcenol | (2,6-dimethyl-7-octen-2-ol) |
| Geraniol | (3,7-dimethyl-*trans*-2,6-octadien-1-ol) |
| Nerol | (3,7-dimethyl-*cis*-2,6-octadien-1-ol) |
| Linalool | (3,7-dimethyl-1,6-octadien-3-ol) |
| Myrcenol | (2-methyl-6-methylene-7-octen-2-ol) |
| Lavandulol | |
| Citronellol | (3,7-dimethyl-6-octen-1-ol) |
| *Trans-trans*-Farnesol | (3,7,11-trimethyl-2,6,10-dodecatrien-1-ol) |
| *Trans*-Nerolidol | (3,7,11-trimethyl-1,6,10-dodecatrien-3-ol) |

| | |
|---|---|
| ALDEHYDES AND ACETALS | |
| Citral | (3,7-dimethyl-2,6-octadien-1-al) |
| Citral diethyl | acetal(3,7-dimethyl-2,6-octadien-1-al diethyl |
| acetal) | |
| Citronellal | (3,7-dimethyl-6-octen-1-al) |
| Citronellyloxyacetaldehyde | |
| 2,6,10-Trimethyl-9-undecenal | |

| | |
|---|---|
| KETONES | |
| Tagetone | |
| Solanone | |
| Geranylacetone | (6,10-dimethyl-5,9-undecadien-2-one) |

| | |
|---|---|
| ACIDS AND ESTERS | |
| *Cis*-Geranic acid | |
| Citronellic acid | |
| Geranyl Esters, including Geranyl formate, Geranyl acetate, Geranyl propionate, Geranyl isobutyrate, Geranyl isovalerate | |
| Neryl Esters, including Neryl acetate | |
| Linalyl Esters, including Lynalyl formate, Linalyl acetate, Linalyl propionate, Linalyl butyrate, Linalyl isobutyrate, | |
| Lavandulyl Esters, including Lavendulyl acetate | |
| Citronellyl Esters, including Citronellyl formate, Citronellyl acetate, Citronellyl propionate, Citronellyl isobutyrate, Citronellyl isovalerate, Citronellyl tiglate | |

| NITROGEN CONTAINING UNSATURATED TERPENE DERIVATIVES | |
|---|---|
| *Cis*-Geranic acid nitrile | |
| Citronellic acid nitrile | |

**TABLE 2. Cyclic Terpenoids**

| ALCOHOLS AND ETHERS | |
|---|---|
| (+)-Neoiso-isopulegol | |
| Isopulegol | (8-*p*-menten-3-ol) |
| Alpha-Terpineol | (1-*p*-menten-8-ol) |
| Beta-Terpineol | |
| Gamma-Terpineol | |
| Delta-Terpineol | |
| 1-Terpinen-4-ol | (1-*p*-menten-4-ol) |

| | |
|---|---|
| ALDEHYDES AND KETONES | |
| Carvone | (1,8-*p*-mantadien-6-one) |
| Alpha-Ionone | (C₁₃H₂₀O) |
| Beta-Ionone | (C₁₃H₂₀O) |
| Gamma-Ionone | (C₁₃H₂₀O) |
| Irone, alpha-, beta-, gamma- | (C₁₄H₂₂O) |
| *n*-Methylionone, alpha-, | beta-, gamma- (C₁₄H₂₂O) |
| Isomethylionone, alpha-, | beta-, gamma- (C₁₄H₂₂O) |
| Allylionone | (C₁₆H₂₄O) |
| Pseudoionone | |
| *n*-Methylpseudoionone | |
| Isomethylpseudoionone | |
| | |
| Damascones | 1-(2,6,6-trimethylcyclohexenyl)-2-buten-1-ones |
| Including beta-Damascenone | 1-(2,6,6-trimethyl-1,3-cyclohadienyl)-2-buten-1-one |
| | |
| Nootkatone | 5,6-dimethyl-8-isopropenylbicyclo [4.4.0]-1-decen-3-one |
| | |
| Cedryl methyl ketone | (C₁₇H₂₆O) |

| | |
|---|---|
| ESTERS | |
| | |
| Alpha-Terpinyl acetate | (1-*p*-menthen-8-yl acetate) |
| | |
| Nopyl acetate | (-)-2-(6,6-dimethylbicyclo [3.1.1]hept-2-en-2-yl)ethyl acetate |
| | |
| Khusymil acetate | |

**Table 3. Cycloaliphatic Compounds Structurally Related to Terpenes**

| ALCOHOLS | |
|---|---|
| 5-(2,2,3-Trimethyl-3-cyclopenten-1-yl)-3-methylpentan-2-ol | |

| | |
|---|---|
| ALDEHYDES | |
| 2,4-Dimethyl-3-cyclohexene carboxaldehyde | |
| 4-(4-Methyl-3-penten-1-yl)-3-cyclohexene carboxaldehyde | |
| 4-(4-Hydroxy-4-methypentyl)-3-cyclohexene carboxaldehyde | |

| | |
|---|---|
| KETONES | |
| Civetone | |
| Dihydrojasmone | (3-methyl-2-pentyl-2-cyclopenten-1-one) |
| Cis-Jasmone | 3-methyl-2-(2-*cis*-penten-1-yl)-2- |
| cyclopenten-1-one | |
| 5-Cyclohexadecen-1-one | |
| 2,3,8,8-Tetramethyl-1,2,3,4,5,6,7,8-octahydro-2-napthalenyl | |
| methyl ketone | |
| 3-methyl-2-cyclopenten-2-ol-1-one | |

| | |
|---|---|
| ESTERS | |
| 4,7-Methano-3a,4,5,6,7,7a-hexahydro-5-(or 6)-indenyl acetate | |
| Allyl 3-cyclohexylpropionate | |
| Methyl dihydrojasmonate methyl (3-oxo-2-pentylcyclopentyl)acetate | |

Amounts of the fragrance may range from about 0.001 to about 10%, preferably from about 0.1 to about 5%, optimally from about 0.5 to about 1% by weight of the personal care composition. The amount of hydrocarbon cyclic terpenoids in the fragrance may range from about 0.000001 to about 90%, preferably from about 0.0001 to about 70%, optimally from about 0.1 to about 30% by weight of the fragrance.

Fragrances of the present invention advantageously will have terpenoid ingredients with boiling points of 250°C or lower. In some instances a fragrance is required to be an enduring one. In those circumstances, the fragrance composition preferably has at least about 40% of the terpenoids that exhibit a ClogP equal or greater than 3.0 and a boiling point equal or greater than 250°C. See U.S. Patent 5,540,853 (Trinh et al.) with regard to definitions of these parameters.

Compositions of this invention will also include a cosmetically acceptable carrier. Amounts of the carrier may range from about 1 to about 99.9%, preferably from about 70 to about 95%, optimally from about 80 to about 90% by weight of the composition. Among the useful carriers are water, emollients, fatty acids, fatty alcohols, humectants, thickeners and combinations thereof. The carrier may be aqueous, anhydrous or an emulsion. Preferably the compositions are aqueous, especially water and oil emulsions of the W/O or O/W or triplex W/O/W variety. Water when present may be in amounts ranging from about 5 to about 95%, preferably from about 20 to about 70%, optimally from about 35 to about 60% by weight.

Emollient materials may serve as cosmetically acceptable carriers. These may be in the form of silicone oils, synthetic esters and hydrocarbons. Amounts of the emollients may range anywhere from about 0.1 to about 95%, preferably between about 1 and about 50% by weight of the composition.

Silicone oils may be divided into the volatile and nonvolatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Nonvolatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about 5 x 10⁻⁶ to 0.1 m²/s at 25°C. Among the preferred nonvolatile emollients useful in the present compositions are the polydimethyl siloxanes having viscosities from about 1 x 10⁻⁵ to about 4 x 10⁻⁴ m²/s at 25°C.

Another class of nonvolatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the ester emollients are:
1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
2) Ether-esters such as fatty acid esters of ethoxylated fatty alcohols.
3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
4) Wax esters such as beeswax, spermaceti wax and tribehenin wax.
5) Sterols esters, of which cholesterol fatty acid esters are examples thereof.
6) Sugar ester of fatty acids such as sucrose polybehenate and sucrose polycottonseedate.

Hydrocarbons which are suitable cosmetically acceptable carriers include petrolatum, mineral oil, C₁₁-C₁₃ isoparaffins, polyalphaolefins, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, hydroxystearic, oleic, linoleic, ricinoleic, arachidic, behenic and erucic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5 to 50%, preferably between 1 and 15% by weight of the composition.

Thickeners can be utilized as part of the cosmetically acceptable carrier of compositions according to the present invention. Typical thickeners include crosslinked acrylates (e.g. Carbopol 982^{®}), hydrophobically-modified acrylates (e.g. Carbopol 1382^{®}), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, and silicates such as magnesium aluminum silicate (Veegum^{®}). Amounts of the thickener may range from 0.0001 to 10%, usually from 0.001 to 1%, optimally from 0.01 to 0.5% by weight of the composition.

Personal care compositions of the present invention may be in any form. These forms may include lotions, creams, roll-on formulations, sticks, mousses, aerosol and non-aerosol sprays and fabric (e.g. nonwoven textile)-applied formulations.

Surfactants may also be present in compositions of the present invention. Total concentration of the surfactant when present may range from about 0.1 to about 40%, preferably from about 1 to about 20%, optimally from about 1 to about 5% by weight of the composition. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionate, C₈-C₂₀ alkyl ether phosphates, C₈-C₂₀ sarcosinates and combinations thereof.

Sunscreen actives may also be included in compositions of the present invention. Particularly preferred are such materials as ethylhexyl p-methoxycinnamate, available as Parsol MCX^{®}, Avobenzene, available as Parsol 1789^{®} and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.1 to 30%, preferably from 2 to 20%, optimally from 4 to 10% by weight of the composition.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may include vitamins. Illustrative vitamins are Vitamin A (retinol), Vitamin B₂, Vitamin B₆, Vitamin C, Vitamin E and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001 to 10%, preferably from 0.01% to 1%, optimally from 0.1 to 0.5% by weight of the composition.

Another type of useful substance can be that of an enzyme such as oxidases, proteases, lipases and combinations. Particularly preferred is superoxide dismutase, commercially available as Biocell SOD from the Brooks Company, USA.

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof. Amounts of these agents may range from about 0.1 to about 10%, preferably from about 0.5 to about 2% by weight of the composition.

Desquamation promoters may be present. Illustrative are the alpha-hydroxycarboxylic acids and beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from about 0.01 to about 15% by weight of the composition.

A variety of herbal extracts may optionally be included in compositions of this invention. Illustrative are green tea, chamomile, licorice and extract combinations thereof. The extracts may either be water soluble or water-insoluble carried in a solvent which respectively is hydrophilic or hydrophobic. Water and ethanol are the preferred extract solvents.

Also included may be such materials as lipoic acid, retinoxytrimethylsilane (available from Clariant Corp. under the Silcare 1M-75 trademark), dehydroepiandrosterone (DHEA) and combinations thereof. Ceramides (including Ceramide 1, Ceramide 3, Ceramide 3B and Ceramide 6) as well as pseudoceramides may also be utilized for many compositions of the present invention but may also be excluded. Amounts of these materials may range from about 0.000001 to about 10%, preferably from about 0.0001 to about 1% by weight of the composition.

Colorants, fragrances, opacifiers and abrasives may also be included in compositions of the present invention. Each of these substances may range from about 0.05 to about 5%, preferably between 0.1 and 3% by weight of the composition.

Except in the operating and comparative examples, or where otherwise explicitly indicated, all numbers in this description indicating amounts of material ought to be understood as modified by the word "about".

The following examples will more fully illustrate personal care compositions comprising terpenoids within and outside the scope of the present invention. All parts, percentages and proportions referred to herein and in the appended claims are by weight unless otherwise illustrated.

### EXAMPLE 1

A series of fragrances containing one or more terpenoids are described below.

| Fragrance A | |
|---|---|
| Ingredient | Weight % |
| Coumarin | 0.5 |
| Benzyl Acetate Extra | 4.0 |
| Benzyl Salicylate | 10.0 |
| Dihydromyrcenol | 10.0 |
| Citronnellol | 10.0 |
| Methyl Cedryl Ketone | 8.0 |
| Methyl Dihydrojasmonate | 5.0 |
| 2-Phenylethanol | 10.0 |
| 5-Acetyl-3-isopropyl-1,1,2,6-Tetramethylindane | 7.5 |
| Jasmopyrane Forte | 10.0 |
| Linalool | 10.0 |
| Alpha-Hexylcinnamic Aldehyde | 8.0 |
| Isolongifolanone | 3.0 |
| Styrallyl Acetate | 1.0 |
| Methylionone | 3.0 |

| Fragrance B | |
|---|---|
| Ingredient | Weight % |
| 1,3,4,6,7,8-Hexahydro-4,6,6,7,8,8-Hexamethylcyclopenta(g)-2-Benzopyran | 7.50 |
| Methyl Cedryl Ketone | 12.00 |
| Lilial | 7.00 |
| Benzyl Salicylate | 7.20 |
| Alpha-Hexylcinnamic Aldehyde | 13.00 |
| Methyl Dihydrojasmonate | 14.60 |
| 2-Phenylethanol | 9.00 |
| Dipropylene Glycol | 18.00 |
| Litsea Cubeba Oil | 5.00 |
| Coumarin | 0.06 |
| Linalool | 3.60 |
| Hexyl Salicylate | 3.04 |

| Fragrance C | |
|---|---|
| Ingredient | Weight % |
| Benzyl Salicylate | 6.5 |
| P-tert Butylanisole | 5.0 |
| Hexyl Salicylate | 4.5 |
| P-tert Butylcyclohexyl Acetate | 12.0 |
| O-tert Butylcyclohexyl Acetate | 3.5 |
| 4-Acetoxy-3-pentyltetrahydropyran | 8.5 |
| 2-Phenylethanol | 7.5 |
| Phenylethyl Acetate | 0.5 |
| Alpha-Hexylcinnamic Aldehyde | 5.0 |
| 2,4-Dimethyl-3-Cyclohexene-1-Carboxaldehyde | 1.5 |
| Methyl Cedryl Ketone | 5.0 |
| 5-Acetyl-3-isopropyl-1,1,2,6-Tetramethylindane | 2.0 |
| 2-Hexyl-2-cyclopentenone | 3.3 |
| Coumarin | 2.0 |
| Hexyl Benzoate | 6.7 |
| Ethyl Cinnamate | 1.5 |
| Diethyl Phthalate | 15.0 |

| Fragrance D | |
|---|---|
| Ingredient | Weight % |
| Cineole | 15.0 |
| Borneol | 10.0 |
| Cedar Wood Oil | 18.0 |
| Clove Terpenese | 2.0 |
| Pine Oil American | 10.0 |
| Diphenyl Oxide | 1.0 |
| Tetrahydrolinalool | 6.0 |
| Fenchyl Acetate . | 5.0 |
| Benzyl Benzoate | 15.0 |
| Isobornyl Acetate | 18.0 |

| Fragrance E | |
|---|---|
| Ingredient | Weight % |
| Anethole | 7.05 |
| Carvone Laevo | 8.90 |
| Menthol Laevo | 10.00 |
| Peppermint Oil American | 23.00 |
| Spearmint Oil American | 51.00 |
| Vanillin | 0.05 |

| Fragrance F | |
|---|---|
| Ingredient | Weight % |
| Benzyl Salicylate | 20 |
| Ethylene Brassylate | 20 |
| Galaxolide - 50%* | 20 |
| Hexyl Cinnamic Aldehyde | 20 |
| Tetrahydro Linalool | 20 |

| | |
|---|---|
| *A 50% solution in benzyl benzoate containing about 80% of enduring perfume components having BP>250°C and ClogP> 3.0. | |

| Fragrance G | |
|---|---|
| Ingredient | Weight % |
| Benzyl Acetate | 4 |
| Benzyl Salicylate | 12 |
| Coumarin | 5 |
| Ethylene Brassylate | 10 |
| Galaxolide - 50%* | 10 |
| Hexyl Cinnamic Aldehyde | 20 |
| Lilial | 15 |
| Methyl Dihydro Isojasmonate | 5 |
| Gamma-n-Methyl Ionone | 10 |
| Patchouli Alcohol | 4 |
| Tetrahydro Linalool | 6 |

| | |
|---|---|
| *Used as a 50% solution in isopropyl myristate containing about 86% of enduring perfume components having BP>250°C and ClogP> 3.0. | |

| Fragrance H | |
|---|---|
| Ingredient | Weight % |
| Benzyl Acetate | 20 |
| Laevo-Carvone | 20 |
| Dihydro Myrcenol | 20 |
| Hydroxycitronellal | 20 |
| Phenyl Ethyl Alcohol | 20 |

| Fragrance I | |
|---|---|
| Ingredient | Weight % |
| Eugenol | 20 |
| Iso-Eugenol | 20 |
| Fenchyl Alcohol | 20 |
| Methyl Dihydrojasmonoate | 20 |
| Vanillin | 20 |

| Fragrance J | |
|---|---|
| Ingredient | Weight % |
| Iso-Bornyl Acetate | 20 |
| Para-Cymene | 20 |
| D-Limonene | 20 |
| Gamma-n-Methyl Ionone | 20 |
| Tetrahydromyrcenol | 20 |

### EXAMPLE 2

A typical cosmetic cream according to the present invention is outlined under Table I.

**TABLE I**

| INGREDIENT | WEIGHT % |
|---|---|
| PHASE A | |
| Water | Balance |
| Disodium EDTA | 0.05 |
| Methyl Paraben | 0.15 |
| Magnesium Aluminum Silicate | 0.60 |
| Triethanolamine | 1.20 |

| PHASE B | |
|---|---|
| Xanthan Gum | 0.20 |
| Natrosol^{®} 250HHR (ethyl cellulose) | 0.50 |
| Butylene Glycol | 3.00 |
| Glycerin | 2.00 |

| PHASE C | |
|---|---|
| Sodium Stearoyl Lactylate | 0.10 |
| Glycerol Monostearate | 1.50 |
| Stearyl Alcohol | 1.50 |
| Isostearyl Palmitate | 3.00 |
| Silicone Fluid | 1.00 |
| Cholesterol | 0.25 |
| Sorbitan Stearate | 1.00 |
| Butylated Hydroxy Toluene | 0.05 |
| Vitamin E Acetate | 0.01 |
| PEG-100 Stearate | 2.00 |
| Stearic Acid | 3.00 |
| Propyl Paraben | 0.10 |
| Parsol MCX^{®} | 2.00 |
| Caprylic/Capric Triglyceride | 0.50 |
| Hydroxycaprylic Acid | 0.01 |
| C12-15 Alkyl Octanoate | 3.00 |

| PHASE D | |
|---|---|
| Malonate Salt | 3.00 |

| PHASE E | |
|---|---|
| Vitamin A Palmitate | 0.10 |
| Bisabolol | 0.01 |
| Vitamin A Acetate | 0.01 |
| Fragrance A | 0.03 |
| Retinol 50C | 0.02 |

### EXAMPLE 3

A water-in-oil topical liquid make-up foundation utilizing the malonate salts of the present invention is described in Table II below.

**TABLE II**

| INGREDIENT | WEIGHT % |
|---|---|
| PHASE A | |
| Cyclomethicone | 9.25 |
| Cetyl Octanoate | 2.00 |
| Dimethicone Copolyol | 20.00 |

| PHASE B | |
|---|---|
| Talc | 3.38 |
| Pigment (Iron Oxides) | 10.51 |
| Spheron L-1500 (Silica) | 0.50 |

| PHASE C | |
|---|---|
| Synthetic Wax Durachem 0602 | 0.10 |
| Arachidyl Behenate | 0.30 |

| PHASE D | |
|---|---|
| Cyclomethicone | 1.00 |
| Trihydroxystearin | 0.30 |

| PHASE E | |
|---|---|
| Laureth-7 | 0.50 |
| Propyl Paraben | 0.25 |

| PHASE F | |
|---|---|
| Fragrance B | 1.00 |

| PHASE G | |
|---|---|
| Water | balance |
| Ammonium Malonate | 3.00 |
| Methyl Paraben | 0.12 |
| Propylene Glycol | 8.00 |
| Niacinamide | 4.00 |
| Glycerin | 3.00 |
| Sodium Chloride | 2.00 |
| Sodium Dehydroacetate | 0.30 |

### EXAMPLE 4

Illustrated herein is a skin cream incorporating the malonate salts of the present invention.

**TABLE III**

| INGREDIENT | WEIGHT % |
|---|---|
| Glycerin | 6.93 |
| Niacinamide | 5.00 |
| Dimethylethanolammonium Malonate | 5.00 |
| Permethyl 101A¹ | 3.00 |
| Sepigel 305² | 2.50 |
| Q2-1403³ | 2.00 |
| Isopropyl Isostearate | 1.33 |
| Arlatone 2121⁴ | 1.00 |
| Fragrance C | 1.00 |
| Cetyl Alcohol CO-1695 | 0.72 |
| SEFA Cottonate⁵ | 0.67 |
| Tocopherol Acetate | 0.50 |
| Panthenol | 0.50 |
| Stearyl Alcohol | 0.48 |
| Titanium Dioxide | 0.40 |
| Disodium EDTA | 0.10 |
| Glydant Plus⁶ | 0.10 |
| PEG-100 Stearate | 0.10 |
| Stearic Acid | 0.10 |
| Purified Water | Balance |

| | |
|---|---|
| ¹ Isohexadecane, Presperse Inc., South Plainfield, NJ ² Polyacrylamide(and)C13-14 Isoparaffin(and) Laureth-7, Seppic Corporation, Fairfield, NJ ³ dimethicone(and)dimethiconol, Dow Corning Corp. Midland, MI ⁴ Sorbitan Monostearate and Sucrococoate, ICI Americas Inc., Wilmington, DE ⁵ Sucrose ester of fatty acid ⁶ DMDM Hydantoin (and) Iodopropynyl Butylcarbamate, Lonza Inc., Fairlawn, NJ | |

### EXAMPLE 5

Illustrative of a powdered cosmetic composition according to the present invention is the formula of Table IV.

**TABLE IV**

| INGREDIENT | WEIGHT % |
|---|---|
| Polysilicone-11 | 27 |
| Cyclomethicone | 54 |
| Petrolatum | 11 |
| Tris(hydroxymethyl)methaneammonium Malonate | 7 |
| Dimethicone Copolyol | 0.5 |
| Fragrance D | 0.5 |

### EXAMPLE 6

A relatively anhydrous composition according to the present invention is reported in Table V.

**TABLE V**

| INGREDIENT | WEIGHT % |
|---|---|
| Cyclomethicone | 79.65 |
| Dimethicone | 9.60 |
| Squalane | 6.00 |
| Isostearic Acid | 1.90 |
| Fragrance E | 1.00 |
| Borage Seed Oil | 0.90 |
| Lithium Malonate (50% in water) | 0.50 |
| Retinyl Palmitate | 0.25 |
| Ceramide 6 | 0.10 |
| Tocopherol | 0.10 |

### EXAMPLE 7

An aerosol packaged foaming cleanser suitable for the present invention is outlined in Table VI.

**TABLE VI**

| INGREDIENT | WEIGHT % |
|---|---|
| Sunflower Seed Oil | 20.00 |
| Maleated Soybean Oil | 5.00 |
| Silicone Urethane | 1.00 |
| Polyglycero-4 Oleate | 1.00 |
| Sodium C14-16 Olefin Sulfonate | 15.00 |
| Sodium Lauryl Ether Sulphate (25% active) | 15.00 |
| Cocoamidopropylbetaine | 15.00 |
| DC 1784^{®} (Silicone Emulsion 50%) | 5.00 |
| Polyquaternium-11 | 1.00 |
| Bis(dimethylethanolammonium) Malonate | 1.00 |
| Fragrance F | 1.00 |
| Water | Balance |

An aerosol is prepared using 92% by weight of the concentrate in Table VI and 8% propellant, the latter being a combination of dimethylether, isobutane and propane.

### EXAMPLE 8

An adhesive cosmetic patch may also be formulated according to the present invention. An adhesive hydrogel is prepared by mixing 30 grams of 2-acrylamido-2-methylpropane sulphonic acid monomer in 20 grams distilled water and 5 grams of a 1% aqueous solution of methylene-bis-acrylamide. The solution is then activated with 0.4% magnesium persulphate catalyst. Shortly after mixing the catalyst with the hydrogel solution, 0.1 grams ammonium malonate in 5 ml water is added. The resultant solution is coated onto a 50/50 blend of polypropylene and hydrophilic polyester and allowed to solidify. The resulting deposited hydrogel is warmed for 24 hours at 40°C in a hot air oven. Final water content of the hydrogel is 50%. Fragrance G is then sprayed on the cooled hydrogel. A polystyrene backing layer is laid over the adhesive hydrogel.

### EXAMPLE 9

A disposable, single use personal towelette product is described according to the present invention. A 70/30 polyester/rayon nonwoven towelette is prepared with a weight of 1.8 grams and dimensions of 15 cm by 20 cm. Onto this towelette is impregnated a composition as outlined in Table VII below.

**TABLE VII**

| INGREDIENT | WEIGHT % |
|---|---|
| Magnesium Malonate | 7.50 |
| Glycerin | 2.00 |
| Hexylene Glycol | 2.00 |
| Disodium Capryl Amphodiacetate | 1.00 |
| Gluconolactone | 0.90 |
| Silicone Microemulsion | 0.85 |
| Witch Hazel | 0.50 |
| PEG-40 Hydrogenated Castor Oil | 0.50 |
| Fragrance H | 0.20 |
| Vitamin E Acetate | 0.001 |
| Water | Balance |

### EXAMPLE 10

The following experiment was conducted to evaluate the effect of a malonate salt (such as DMAE malonate) to enhance the oxidative stability of cinnamaldehyde, a typical fragrance ingredient. Cinnamaldahyde (0.025 ml) was mixed with methanol (15.98 ml). This solution (3 ml) was aliquoted into a cuvette and placed in a UV spectrophotometer (Perkin-Elmer Lambda 35 instrument). The spectrophotometer was operated in the range 240-450 nm to obtain an absorption spectrum versus a methanol control solution (Spectrum A). A second cinnamaldahyde (0.025 ml) charge was mixed with methanol (15.98 ml). Hydrogen peroxide (0.045 g) was added to this solution with mixing for 30 seconds. Thereafter 3 ml of the solution was aliquoted into a cuvette and placed in the UV spectrophotometer. The range of 240-450 nm of the spectrophotometer was scanned to obtain an absorption spectrum versus a methanol control solution (Spectrum B).

A third solution was blended. This consisted of cinnamaldahyde (0.025 ml) and mixed with methanol (15 ml) and dimethylaminoethanol salt of malonic acid (DMAE malonate) (1 ml). Hydrogen peroxide (0.045 g) was added to this solution and allowed to mix for 30 seconds. A portion of the solution (3 ml) was aliquoted into a cuvette and placed for measurement in the UV spectrophotometer. Again the range 240-450 nm of the spectrophotometer was scanned to obtain an absorption spectrum versus a methanol control solution (Spectrum C).

Spectrum A corresponding to cinnamaldahyde exhibited an absorption maximum around 287 nm. Spectrum B obtained upon adding hydrogen peroxide to cinnamaldahyde (to enhance oxidative decomposition), exhibited a significant increase in the intensity of the absorption maximum at 287 nm. Specifically, the ratio at 287 mn of the absorptions for Spectrum B to Spectrum A was 1.4. The increase in absorption intensity at this wavelength can be attributed to an increase in the species absorbing at that wavelength, due to oxidation induced by hydrogen peroxide.

Spectrum C obtained upon addition of hydrogen peroxide to cinnamaldahyde in the presence of DMAE malonate showed a decrease in the intensity of the absorption maximum at 287 nm relative to Spectrum B. The absorption intensity ratio of Spectrum C to Spectrum B was 0.4. The result indicates a retardation of the hydrogen peroxide induced oxidation of cinnamaldahyde. Accordingly, it is evident that malonate salts do have a stability enhancing effect upon fragrance components.

## Claims

1. A personal care composition comprising:
(i) from 0.001 to 10% of a fragrance by weight of the composition comprising from 0.000001 to 90% of a hydrocarbon cyclic terpenoid by weight of the fragrance;
(ii) from 0.0001 to 30% by weight of the composition of a salt of malonic acid; and
(iii) from 1 to 99% by weight of the composition of a cosmetically acceptable carrier.

2. A composition according to claim 1 wherein the hydrocarbon cyclic terpenoid is selected from the group consisting of limonene, alpha-terpinene, gamma-terpinene, alpha-pinene and beta-pinene.

3. A composition according to claim 1 or claim 2 wherein the malonic acid is present as a half neutralized and a fully neutralized acid in a molar ratio ranging from 1000:1 to 1:1000, respectively.

4. A composition according to claim 3 wherein the molar ratio is 2:1 to 1:200.

5. The composition according to any one of the preceding claims wherein the salt has a cationic counterion to malonate which is an inorganic cation selected from the group consisting of lithium, sodium, potassium, magnesium, calcium, ammonium and combinations thereof.

6. The composition according to any one of claims 1 to 4 wherein the salt has a cationic counterion to malonate which is an organic cation having from 2 to 1,000 carbon atoms selected from the group consisting of polyethyleneimine, triethanolamine, diethanolamine, propanolamine, monoethanolamine, methylamine, ethylamine, propylamine, isopropylamine, butylamine, isobutylamine, t-butylamine, pentylamine, isopentylamine, hexylamine, cyclohexylamine, cyclopentylamine, norbornylamine, octylamine, ethylhexylamine, nonylamine, decylamine, pyrrolidone, amino adds, 2-emino-2-methyl-1-propanol, dimethylethanolamine, tris(hydroxymethyl)amino methane and combinations thereof.

## Patentansprüche

1. Körperpflegemittel, umfassend:
(i) 0,001 bis 10 Gewichts-% eines Duftstoffs, bezogen auf die Zusammensetzung, umfassend 0,000001 bis 90 Gewichts-% eines cyclischen Terpenoidkohlenwasserstoffs, bezogen auf den Duftstoff;
(ii) 0,0001 bis 30 Gewichts-% eines Malonsäuresalzes, bezogen auf die Zusammensetzung, und
(iii) 1 bis 99 Gewichts-% eines kosmetisch verträglichen Trägers, bezogen auf die Zusammensetzung.

2. Mittel gemäß Anspruch 1, wobei der cyclische Terpenoidkohlenwasserstoff ausgewählt ist aus der Gruppe, bestehend aus Limonen, alpha-Terpinen, gamma-Terpinen, alpha-Pinen und beta-Pinen.

3. Mittel gemäß Anspruch 1 oder Anspruch 2, wobei die Malonsäure als eine halbneutralisierte und eine vollständig neutralisierte Säure in einem Molverhältnis im Bereich von 1000:1 bis 1:1000 vorliegt.

4. Mittel gemäß Anspruch 3, wobei das Molverhältnis 2:1 bis 1:200 ist.

5. Mittel gemäß einem der vorangehenden Ansprüche, wobei das Salz ein kationisches Gegenion zu Malonat hat, welches ein anorganisches Kation ist, ausgewählt aus der Gruppe, bestehend aus Lithium, Natrium, Kalium, Magnesium, Calcium, Ammonium und Kombinationen davon.

6. Mittel gemäß einem der Ansprüche 1 bis 4, wobei das Salz ein kationisches Gegenion zu Malonat hat, welches ein organisches Kation mit 2 bis 1000 Kohlenstoffatomen ist, ausgewählt aus der Gruppe, bestehend aus Polyethylenimin, Triethanolamin, Diethanolamin, Propanolamin, Monoethanolamin, Methylamin, Ethylamin, Propylamin, Isopropylamin, Butylamin, Isobutylamin, t-Butylamin, Pentylamin, Isopentylamin, Hexylamin, Cyclohexylamin, Cyclopentylamin, Norbornylamin, Octylamin, Ethylhexylamin, Nonylamin, Decylamin, Pyrrolidon, Aminosäuren, 2-Amino-2-methyl-1-propanol, Dimethylethanolamin, Tris(hydroxymethyl)aminomethan und Kombinationen davon.

## Revendications

1. Composition de soin personnel comprenant :
(i) de 0,001 à 10 % en poids d'un parfum, par rapport à la composition, comprenant de 0,000001 à 90 % en poids d'un terpénoïde hydrocarboné cyclique par rapport au parfum ;
(ii) de 0,0001 à 30 % en poids d'un sel d'acide malonique, par rapport à la composition ; et
(iii) de 1 à 99 % en poids d'un véhicule acceptable au plan cosmétique, par rapport à la composition.

2. Composition selon la revendication 1, dans laquelle le terpénoïde hydrocarboné cyclique est choisi dans le groupe constitué par le limonène, l'alpha-terpinène, le gamma-terpinène, l'alphapinène et le bêta-pinène.

3. Composition selon la revendication 1 ou la revendication 2, dans laquelle l'acide malonique est présent sous forme d'un acide à moitié neutralisé et entièrement neutralisé dans un rapport molaire s'échelonnant respectivement de 1000:1 à 1:1000.

4. Composition selon la revendication 3, dans laquelle le rapport molaire est de 2:1 à 1:200.

5. Composition selon l'une quelconque des revendications précédentes, dans laquelle le sel a un contre-ion cationique du malonate qui est un cation inorganique choisi dans le groupe constitué par le lithium, le sodium, le potassium, le magnésium, le calcium, l'ammonium et les combinaisons de ceux-ci.

6. Composition selon l'une quelconque des revendications 1 à 4, dans laquelle le sel a un contre-ion cationique du malonate qui est un cation organique ayant de 2 à 1000 atomes de carbone choisi dans le groupe constitué par la polyéthylèneimine, la triéthanolamine, la diéthanolamine, la propanolamine, la monoéthanolamine, la méthylamine, l'éthylamine, la propylamine, l'isopropylamine, la butylamine, l'isobutylamine, la t-butylamine, la pentylamine, l'isopentylamine, l'hexylamine, la cyclohexylamine, la cyclopentylamine, la norbornylamine, l'octylamine, l'éthylhexylamine, la nonylamine, la décylamine, la pyrrolidone, les acides aminés, le 2-amino-2-méthyl-1-propanol, la diméthyléthanolamine, le tris(hydroxyméthyl)amino-méthane et les combinaisons de ceux-ci.
